# EUROPEAN PATENT APPLICATION

(11) **EP 1 072 223 A1**
(43) Date of publication of application: **31.01.2001**
(21) Application number: 00306354.2
(22) Date of filing: 26.07.2000
(51) Int. Cl.: A61B 6/00

(54) **Marker element**

(30) Priority: 29.07.1999 GB 9917812
(71) Applicant: United Bristol Healthcare NHS Trust, Bristol, BS1 3NU (GB)
(72) Inventor: Goddard, Paul Richard, Redland, Bristol BS6 7HF (GB)
(74) Representative: Beck, Simon Antony

(57) **Abstract**

A marker (20) is provided for aiding spatial correlation between an image, such as a MRI image, and the physical body which was imaged. The marker (20) in one embodiment comprises a plurality of tubes (22-40) of monotonically changing length such that the position of a view slice perpendicular to the axis of the tubes can be determined by counting the number of tubes visible in the view slice.

## Description

The present invention relates to a marker for indicating position and orientation of an image obtained using a diagnostic instrument, for example a non-invasive diagnostic instrument such as a nuclear magnetic resonance imaging (MRI) scanner or a computed tomography (CT) scanner.

MRI scanners are able to provide three dimensional non-invasive data acquisition within the human body. Data for a plurality of voxels (the three dimensional equivalent of a pixel) are stored on a data recorder, and may be used to reconstruct various view slices through the subject. Each voxel is typically a few millimetres from side to side.

The precision of these non-invasive scans allows features within the body, such as tumours, to be localised with great accuracy. This information is crucial if, for example, it is desired to perform a biopsy. However, due to the fact that the patient can move during the scan, and must be removed from the scanner in order to have other procedures performed thereon, it can be difficult to accurately correlate the views received from the scan with the actual position of the patient.

Medical image data is often recorded onto film for subsequent viewing by a specialist. The film, by virtue of its transparent nature, can be viewed from either side and this could result in left/right image conversion. This could result in a procedure being carried out on the wrong side of a patient. In addition patients can be entered into the MRI or CT machine head first or feet first, prone or supine, all giving rise to potential errors of laterality.

According to the present invention, there is provided a marker for indicating position and/or orientation in a medical MRI or CT imaging system, the marker comprising at least one element which has a spatially varying shape and which is observable by the imaging system.

Preferably, the or each marker comprises at least one elongate element which is observable by the imaging system, or which defines a chamber which is filled by or contains a medium which is observable to an imaging system.

In an example of an orientation marker constituting an embodiment of the present invention, an elongate case, for example of plastics, defines a chamber. Advantageously, the chamber exhibits one or zero degrees of mirror symmetry. This is to ensure that, should the image be viewed from the wrong side, the marker will provide sufficient information to determine this fact. The chamber may, for example, define the shape of a letter, for example "R" for right, "L" for left, "a" for anterior, or "p" for posterior. It will be appreciated that other letters may also be chosen depending on language and use. The letter R is particularly preferred since it does not exhibit mirror symmetry. Similarly the marker should preferably exhibit little or no rotational symmetry.

In the context of a positioning device, it is preferable that the interior of the device be effectively divided into a plurality of adjacent chambers, with the chambers of varying length.

The division between adjacent chambers may be total or partial, total division may be achieved by forming the adjacent chambers as individual tubes placed in a side by side manner. However, it will be appreciated that it is sufficient that the change in a cross section be observable between adjacent chambers, in order that the chambers can be distinguished from one another.

Preferably, the chambers change in length in a monatomic fashion with lateral position within the marker device. In a marker constituting an embodiment of the present invention, ten tubes, of different length, are arranged, side by side in a planar manner in order of increasing length. This allows position to be determined to 10 discreet imaging planes.

Advantageously, each chamber may be modified along its longitudinal axis in order to define a plurality of volumes containing different amounts of fluid. The volumes are observable as having different cross sections. In an arrangement in which the volumes have a large cross section or a small, preferably zero, cross section it will be appreciated that this is akin to the "ones" and "zero" in a binary system. Thus by arranging a plurality of changes in a laterally spaced manner, and by varying the cross section of the chambers along their longitudinal direction, it is possible to write a series of binary words which vary in the longitudinal direction. Thus 4 chambers arranged in a side by side manner can be arranged to provide 16 discreet indications of distance along the length.

Advantageously the chambers are filled with water. The water may be doped in order to improve its visibility to the imaging system. Doping with Gadolinium-chelate enhances the visibility to magnetic resonance imaging devices. Iodinated contrast media is particularly visible to CT scanners. Other media include vegetable oils. It is particularly advantageous if the contrast media has little or no toxicity.

A marker device constituting an embodiment of the present invention may be formed as a biopsy plate which may, optionally, include a protractor.

The function of a biopsy plate is to act as a "sight" for placement of the biopsy needle. Thus an essentially two dimensional arrangement may be provided which has co-operating markers such that lines projected from the markers intersect to define the area into which the biopsy needle should be placed. However, it is also possible to define the biopsy site by providing a through hole in the biopsy plate.

Advantageously the biopsy plate is visible to the imaging system. Alternatively the plate may carry or have embedded therein markers which are visible to imaging systems.

The markers may not only serve to make the biopsy plate visible to the imaging system, but may also serve to convey additional information, such as angle with respect to an axis of the biopsy plate. The biopsy needle may have to enter the biopsy site on a non-normal orientation in order to reach a target area. As a consequence it may be necessary to define one or both of azimuthal direction (ie a horizontal angle with respect to a direction) and an inclination with respect to the plane of the biopsy plate.

In order to define the azimuthal direction, one or more angle markers may be defined adjacent the biopsy site. The markers may, for example, be defined by channels of suitably doped fluid formed within or on the surface of the biopsy plate. Channels on the surface of the plate may be defined by closed walls in order to prevent leakage.

Alternatively non-visible marker elements may be placed within a volume of liquid so as to define channels or shadows within the liquid (the liquid being visible to the scan system).

A protractor may be provided in a plane perpendicular to the biopsy plate. The protractor need not have a visible marker element included therein as the angle with respect to the biopsy plate can be calculated from the medical images.

The present invention will further be described, by way of example, with reference to the accompanying drawings, in which:
Figure 1 is a perspective view of a orientation indicator constituting a first embodiment of the present invention;
Figure 2 is a plan view of a position indicator constituting a second embodiment of the present invention;
Figure 3 schematically illustrates the position indicator of Figure 2 as it appears on an MRI scan through a plane perpendicular to the plane of the indicator along the line A-A'; and
Figure 4 is a perspective view of a biopsy needle guide arrangement.

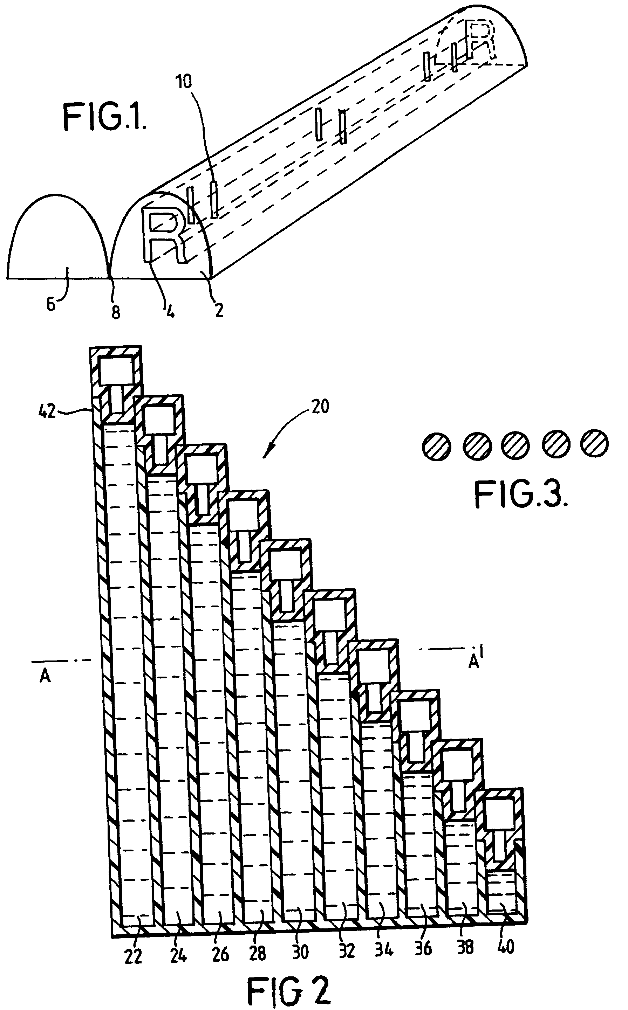

Figure 1 schematically illustrates an orientation marker constituting an embodiment of the present invention, in use, the orientation marker is placed adjacent the patient during a scan, and in a predetermined orientation. The marker illustrated in Figure 1 comprises an elongate plastics tube 2 which has been machined, for example drilled through, to define a letter 4, in this case "R" throughout the length of the tube. The letter 4 is hollow, and is filled with doped water in order to provide a contrast media for MRI scanning. A removable cover 6, which may be attached by a hinge 8, is provided in order that the contrast media can be topped up if necessary. This may become necessary since some plastics absorb water into their structure.

In an alternative arrangement, the letter 4 may be solid, for example of extruded plastics, and may be attached to the casing 2, for example by attachment components 10. In this configuration, the space around the letter is filled with a contrast media and the letter appears as a dark feature surrounded by the contrast media.

The clearly identifiable correct configuration for the mark ensures that images of the patient can be unambiguously interpreted with reference to the marker in order to determine which way round or up the image should be viewed.

Figure 2 schematically illustrates an image localisation device 20. The device comprises a plurality of tubes 22, 24, 26, 28, 30, 32, 34, 36, 38 and 40 arranged adjacent one another in a planar fashion. Each tube 20 - 40 aligns with every other tube at a first end of the device. The tubes are of different lengths, with tube 22 being the longest and tube 40 being the shortest and the tubes being arranged in monatomically decreasing length. Furthermore, each tube varies in length from its neighbour by a predetermined step size. The tubes may be attached to a plastics backing element in order to hold them in this configuration. In the arrangement shown, each tube is filled with a contrast media, for example doped water, vegetable oil or iodinated fluid and then sealed with a stopper 42.

In use, the localisation device is attached to the patient, for example by contact adhesive or a tape, the position of the device may also be indicated on the patient's skin using a marker pen. The scanning is performed in a plane substantially perpendicular to the longitudinal axis of the tubes 22 - 40.

Figure 3 schematically illustrates the appearance of the localisation device in a scan along the line A-A'. This scan line intersects tubes 22, 24, 26, 28, and 30. Thus these five tubes show up as five dots on the scanned image. The scan line does not intersect tubes 34, 36, 38 and 40. Furthermore, the scan line intersects the stopper of tube 32 which is made of a medium which is not visible to the scanning system. Thus it will be appreciated that if the scan line moves along the length of the localisation device, the number of tubes that are visible varies. Once the feature of interest has been located within a medical image, it is merely necessary to count the number of markers visible, and once the patient has been withdrawn from the scanning device, this position can be determined with reference to the physical location of the marker on the patient and a suitable indication made on the patient's skin, for example with a pen. The device may then be repositioned such that all or nearly all of the markers are viewable within the selected scan plane and the patient may then be re-scanned in order that these markers can then serve to indicate the chosen site for entry of a medical instrument, such as a biopsy needle.

It will be appreciated that, more than one marker device may be used in a single scan and that the markers may be arranged in different orientations (given that the medical image itself can be viewed in different orientations).

It will be appreciated that the features of the first embodiment may be combined with the second embodiment such that not only does the device give an indication of longitudinal position along the patient, but it also provides an indication of orientation of the image.

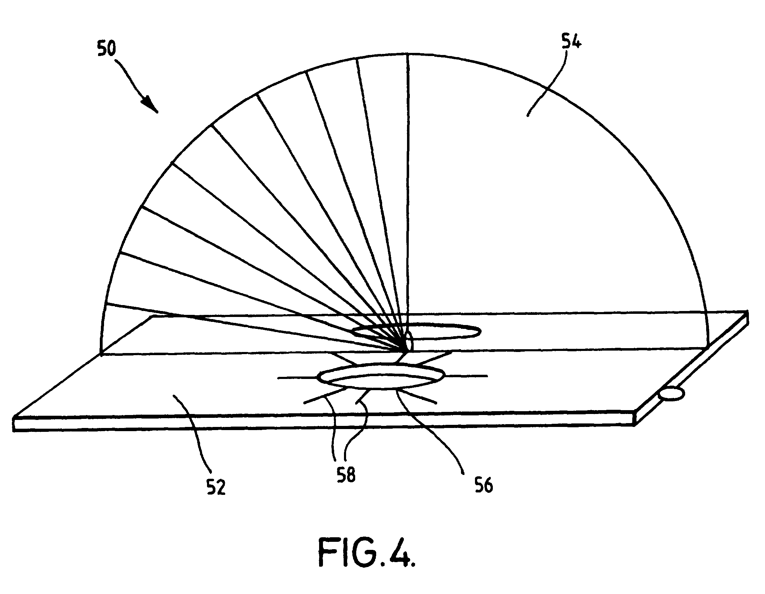

Figure 4 illustrates a biopsy needle guide, generally indicated as 50, which has a base plate 52 and a protractor 54 perpendicular to the plane of the base plate. The base plate 52 has a hole 56 formed therein through which a biopsy needle is, in use, passed. The plate 52 is hollow and contains water doped as described hereinbefore so as to show up n a non-invasive scan. Angle markers 58 are formed in the plate such that the needle direction with, for example, respect to the longitudinal axis of the guide can be measured. The markers may merely be formed on the upper surface of the guide since all the required directional information can be derived from the scanned data. However, the markers may be formed by elements extending into the body of the doped water, thereby becoming visible in the scanned data. As a further alternative the markers may be formed by a fluid containing channels in an otherwise solid or non-fluid containing base plate.

The protractor 54 may be detachable from the base plate 52. This enables the protractor to be replaced if it is damaged. In a simple version of the device, the entirety of the device may be rotated with respect to the patient such that the biopsy needle is adjacent to protractor so the angle of inclination can be read. However, in other versions of the device, the protractor may be held on a rotating base such that the protractor can be rotated with respect to the base plate 52 for ease of use.

Although the use of plastics, such as perspex, has been described it will be appreciated that other materials may be used, as long as they provide sufficient contrast with the contrast media.

It is thus possible to provide a safe and easily usable device for improving the precision of medical imaging and the like.

## Claims

1. A marker for indicating position and/or orientation in a non-invasive imaging system, the marker characterised by comprising at least one element (2, 22, 24, 26, 28, 30, 32, 34, 36, 38, 40) which has a spatially varying shape and which is observable by the imaging system.

2. A marker as claimed in claim 1, characterised in that the marker comprises at least one elongate element (2, 22, 24, 26, 28, 30, 32, 34, 36, 38, 40) which is observable by the imaging system or which defines a chamber which contains a medium which is observable to the imaging system.

3. A marker as claimed in claim 2, characterised in that the marker defines a chamber having one or zero degrees of mirror symmetry.

4. A marker as claimed in claim 2 or 3, characterised in that the marker defines a chamber having one or zero degrees of rotational symmetry.

5. A marker as claimed in any one of the preceding claims, characterised in that the interior of the device is divided into a plurality of chambers.

6. A marker as claimed in claim 5, characterised in that the chambers are of varying length.

7. A marker as claimed in claim 6, characterised in that the length of the chambers vary in a monotonic fashion.

8. A marker as claimed in claim 4, characterised in that the cross-section of the chambers vary as a function of length and lateral position within the marker.

9. A marker as claimed in claim 8, characterised in that the chambers define a code which varies with respect to the longitudinal position along the marker.

10. A biopsy plate comprising a marker as claimed in any one of the preceding claims.

11. A biopsy plate as claimed in claim 10, characterised by including azimuthal markings thereon or therein.

12. A biopsy plate as claimed in claim 10 or 11, further including a protractor.

13. A marker as claimed in any one of the preceding claims having chambers containing doped water, iodinated contrast media or other suitable contrast media therein.
